# EUROPEAN PATENT SPECIFICATION

(11) **EP 2 490 730 B1**
(45) Date of publication and mention of the grant of the patent: **25.02.2015**
(21) Application number: 10773014.5
(22) Date of filing: 18.10.2010
(51) Int. Cl.: A61M 1/16, B01D 61/14

(54) **Medical fluid delivery system**
Medizinisches Flüssigkeitsabgabesystem
Système de délivrance de fluide médical

(30) Priority: 21.10.2009 EP 09013282
(43) Date of publication of application: 29.08.2012
(73) Proprietor: Gambro Lundia AB, 220 10 Lund (SE)
(72) Inventor: KRAUSE, Bernd, 72414 Rangendingen (DE); KIEFERLE, Verena, 72072 Tuebingen (DE); DRIESCHMANNS, Hans-Rainer, 72072 Tuebingen (DE)
(74) Representative: Perchenek, Nils
(86) International application number: PCT/EP2010/065636
(87) International publication number: WO 2011/048052

(56) References cited:
- EP-A1- 1 710 011
- WO-A1-93/22029
- WO-A1-2009/045268
- GB-A- 2 043 478
- US-A- 4 702 829
- US-A- 5 269 931
- US-A- 5 531 893
- US-A- 5 622 626
- US-A1- 2003 146 156
- US-A1- 2005 131 331

## Description

### Technical Field

The present disclosure relates to a medical fluid delivery system for on-line production of sterile medical fluid which is free from cytokine inducing substances (CIS) and a module for a medical fluid delivery system capable of removing cytokine inducing substances (CIS) from a medical fluid as well as a process for the production of sterile medical fluid which is free from cytokine inducing substances (CIS).

### Description of the Related Art

Medical treatments like hemodialysis, hemodiafiltration and hemofiltration require large amounts of sterile medical fluid. During hemo(dia)filtration, patients are exposed to up to 150 1 of substitution fluid, 3 times a week, 52 weeks a year, which leads to a high volume of fluid directly injected into the patient's blood. It is therefore crucial that the fluid is free of harmful inflammatory substances like bacteria and endotoxins.

It has recently been recognized that not only bacteria and endotoxins, but also cytokine inducing substances (CIS), when present in human blood, can cause clinical consequences such as inflammation status and endothelial damage (e.g. Blood Purif 27 (2009) 81-85; Nephrol Dial Transplant 23 (2008) 3635-3642).

State-of-the-art medical fluid delivery systems are capable of producing dialysis fluid for hemofiltration treatments or substitution fluid for hemo(dia)filtration treatments on-line, producing a fluid with 0 CFU/ml and endotoxin concentrations of 0,03 EU/ml and less, but they are not capable of removing cytokine inducing substances (CIS) from the medical fluid.

WO 2008/053259 A1 discloses an apparatus for hemo(dia)-filtration comprising a disposable fluid circuit associable to a machine for on-line preparation of a dialysis fluid and usable for performing a hemodiafiltration treatment with pre-dilution and post-dilution. The replacement fluid supply line of the apparatus comprises an ultrafilter intended to reduce the amount of endotoxins in the fluid. Dialysis fluid produced on-line is either fed directly to the dialysate side of a hemo(dia)filtration device or fed to the replacement fluid supply line of the apparatus and infused into the patient subsequent to ultrafiltration.

US 2005/0131331 A1 discloses medical fluid delivery systems capable of performing pre- and/or post-dilution clearance modes. A medical fluid system is disclosed comprising three filters in series without an intervening pump placed between the filters. to remove bacteria and endotoxins. The first two filters are reusable ultrafilters and the third filter can be a single use microfilter or a reusable or single use ultrafilter.

US 5 269 931 A discloses a microporous cationically charged semihydrophobic polyethersulfone membrane which can be used for filtration of fluids, macromolecular transfers, electrophoresis, blotting methods, and the like.

US 5 531 893 A discloses a hydrophilic charge-modified microporous membrane having a cross-linked structure of an inter-penetrating polymer network. The membrane is used for filtration applications, particularly the filtration of ultrapure water used in the manufacture of computer chips.

EP 1 710 011 A1 discloses an ultrafiltration membrane capable of retaining bacterial DNA and/or bacterial DNA fragments. However, in order to achieve sufficient filtration rates for the high medical fluid volumes mentioned above, a high surface area is required. Therefore, the shape of the membrane has to be modified by complex production technologies such as formation of hollow fibre membranes or pleating of the membranes.

US 2003/0146156 discloses a system for removing protein aggregates from a protein solution, wherein the solution is first passed through a charged microporous membrane and subsequently through an ultrafiltration membrane.

### Summary

It is an object of the present invention to provide a medical fluid delivery system capable of on-line production of a medical fluid, which is sterile and non-pyrogenic and also free from cytokine inducing substances (CIS).

It is also an object of the present invention to provide a single use module for a medical fluid delivery system which is capable of removing cytokine inducing substances (CIS) from a medical fluid.

It is another object of the present invention to provide a process for the production of a sterile medical fluid free from bacteria, endotoxins and cytokine inducing substances (CIS).

It has been found that the incorporation of a positively charged microporous membrane into a system for the on-line production of medical fluid leads to an improved medical fluid, as cytokine inducing substances (CIS) are removed from the fluid.

### Brief Description of the Drawings

Figure 1 shows a schematic view of a medical fluid delivery system according to the invention comprising three filters;
Figure 2 shows a schematic view of another medical fluid delivery system according to the invention comprising two filters;
Figure 3 shows one version of a module for removing cytokine inducing substances (CIS) from a medical fluid according to the invention;
Figure 4 shows another version of a module for removing cytokine inducing substances (CIS) from a medical fluid according to the invention.
Figure 5 shows an exploded view of a single use module according to the invention comprising a filter unit for removing cytokine inducing substances (CIS) from a medical fluid, arterial and venous expansion chambers, pressure transducers, a pump tube tract, and a valve.
Figure 6 shows a perspective view of the base plate of the module shown in Fig. 5.
Figure 7 shows a perspective view of the housing cover of the module shown in Fig. 5.

### Detailed Description

The present invention provides a medical fluid delivery system according to claim 1 and a method for producing a sterile medical fluid according to claim 13.

The medical fluid delivery system of the invention comprises at least two filter modules **(2,3,4)** connected in series. The system comprises at least one ultrafilter and at least one microporous filter, wherein the final filter module in the series is a microporous filter comprising a positively charged microporous flat sheet membrane having a surface area in the range from 8 to 30 cm².

The system of the present invention comprises a combination of ultrafilters and microporous filters. In general, microporous filters differ from ultrafilters in the capability of the different filters in removing small objects. In general, ultrafilters can remove smaller objects than microporous filters.

The term "ultrafilter" as used herein includes filters having a membrane pore or membrane opening diameter or length of about 1 to about 100 nm, which effectively filters objects such as endotoxins, viruses and proteins. In one preferred embodiment, the ultrafilters used in the present invention have pore sizes in the range of from about 1 to about 10 nm.

These ultrafilters usually have a surface area of at least 2.1 m². Commercially available examples of such ultrafilters are Gambro U8000 and U9000 filters. Membranes used for such filters are often based on polysulfone and polyethersulfone. These ultrafilters are multi-use products for medical fluid filtration which can be used for a period of up to several weeks and hence have to withstand certain disinfection cycles and high temperatures during cleaning procedures.

For purposes of the present invention, the term "microporous filter" includes filters having a membrane pore or membrane pore size of at least 0.2 µm, which effectively filters objects, such as yeast, fungi, bacteria and some proteins. In one embodiment, the microporous filters used in the present invention have pore sizes in the range of about 200 to about 400 nm.

According to the invention, the positively charged microporous membrane is a flat sheet membrane **(14)**. Alternatively, the membrane may be a hollow fibre membrane and the microporous filter module may comprise a multitude of microporous positively charged hollow fibre membranes.

An advantage of the positively charged microporous membrane is that it has excellent retention of cytokine inducing substances (CIS) even if the surface area of the membrane is small, and enables the high fluid flow rates required for the on-line production of medical fluid, e.g., substitution fluid for hemo(dia)filtration. This allows the filter module to be miniaturized and dispenses with the need for a holding tank or reservoir for the medical fluid within the system.

In one embodiment, the membrane comprises polyethersulfone. Other options are surface-charged Nylon or cellulose acetate.

The positive charges can be provided by modifying the membrane by adding quaternary ammonium groups to its surface. Modification of the membrane can be achieved by reacting the membrane substrate with a charge modifying agent like polyethyleneimine epichlorohydrin, as shown in US 5269931 A. Modification of the membrane can also be achieved by incorporation of a copolymer comprising quaternary ammonium groups into the membrane substrate as shown in US 5531893 A

An example of a suitable flat sheet membrane is commercially available under the trade name PALL™ HP200.

Examples of medical fluids that can be prepared with a system according to the invention are ultrapure dialysis fluid and non-pyrogenic substitution fluid for hemo(dia)filtration. Other examples are peritoneal dialysis fluids, fluids for laparoscopy, biotechnological applications, such as separation of bacteria cultures from solute synthesis products, and similar uses.

Furthermore, such fluids can be used in chip manufacturing or for the exclusion of particles from process water used for chip manufacturing.

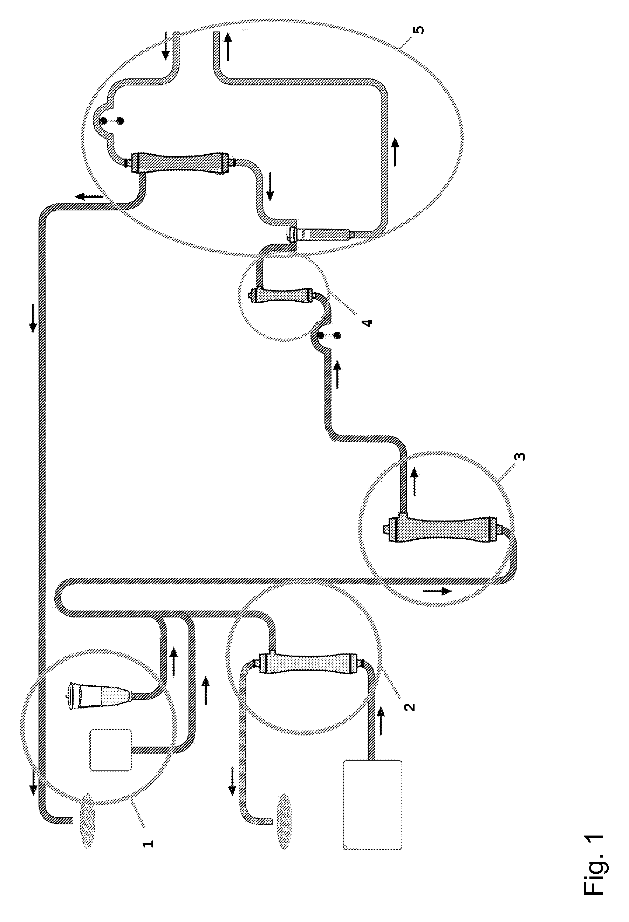

Fig. 1 shows an example of a system according to the invention comprising three filter modules **(2,3,4)** connected in series, the first filter module **(2)** comprising an ultrafilter, the second filter module **(3)** comprising an ultrafilter and the third filter module **(4)** comprising a microporous filter, wherein the microporous filter comprises a positively charged microporous membrane. The third filter module is a sterile single use device and also serves as sterile barrier before the infusion of medical fluid into the patient. Constituents **(1)** can be added to the fluid, for instance dialysis fluid concentrate or electrolytes carbohydrates, drugs, and the like. The medical fluid is prepared by filtering water from a RO (reverse osmosis) unit through a first ultrafilter module **(2),** adding constituents **(1),** and filtering the resulting solution through a second ultrafilter module **(3),** followed by filtration through a third filter module **(4)** comprising a positively charged microporous membrane. The resulting medical fluid then is ready to be administered to the patient, for instance via an extracorporeal blood circuit **(5).**

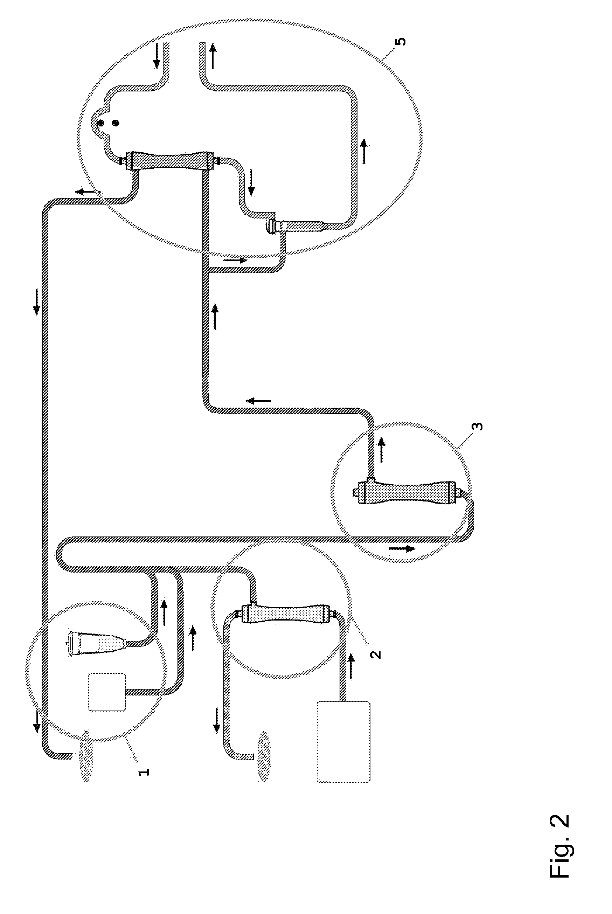

Fig. 2 shows a schematic view of a medical fluid delivery system according to the invention: the medical fluid is prepared by filtering water from a RO (reverse osmosis) unit through a first ultrafilter module **(2),** adding constituents **(1),** and filtering the resulting solution through a second filter module **(3)** comprising a positively charged microporous membrane. The resulting fluid is used as dialysis fluid in an extracorporeal blood circuit **(5).**

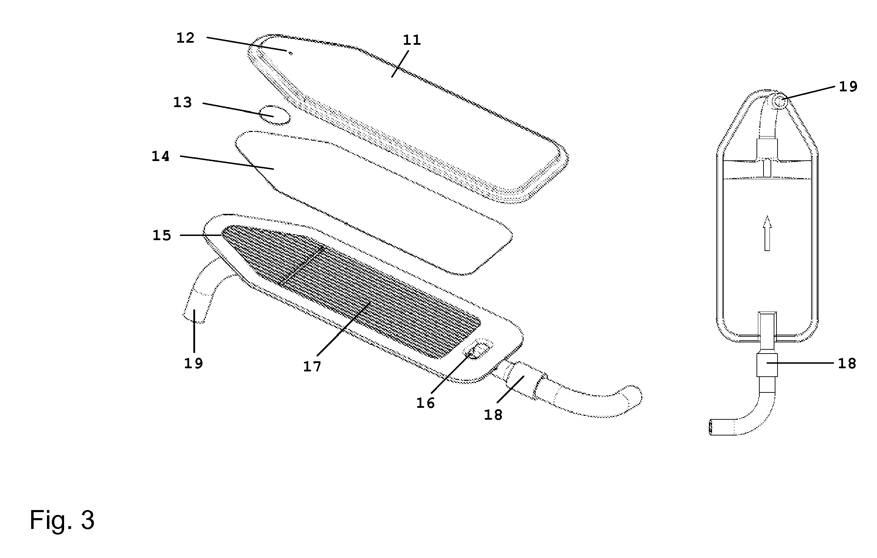

In one embodiment of the invention, the filter module comprising the positively charged microporous membrane is part of a single use module. One embodiment of such a single use module is shown in Fig. 3. In this embodiment, both inlet **(18)** and outlet **(19)** are on the same side of the housing. The fluid enters the module from the bottom part **(15)** of the filter through a deflector **(16),** enters the upper part **(11)** of the module, passes the filtration membrane **(14)** to the bottom part **(15)** and leaves through the outlet **(19).** The filter module comprises a vent hole **(12)** equipped with a deaeration membrane **(13)** on the triangular end of the upper part **(11)** of the housing. When the fluid passes the vent hole **(12),** any entrapped air passes the deaeration membrane **(13)** and is purged through the vent hole **(12).** The membrane support structure **(17)** provides support to the filtration membrane **(14).** It comprises channels to guide the fluid flow along the module and a lateral fluid collector duct.

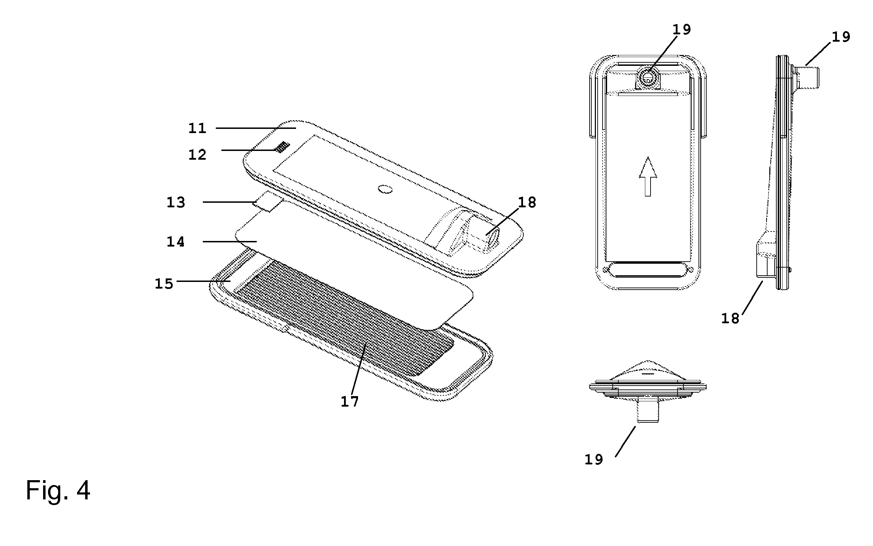

Another embodiment of a single use filter module is shown in Fig. 4. In this embodiment, inlet **(18)** and outlet **(19)** are on different sides of the housing. Fluid enters the filter module from the upper part **(11)** and then is filtered through a filter membrane **(14).** Any entrapped air passes the deaeration membrane **(13)** and is purged through the venting slots **(12).** The bottom part of the housing **(15)** comprises a support structure **(17)** to support the membrane and guide the fluid to the outlet **(19)** and to provide uniform discharge of the fluid.

In one embodiment, the filter module is part of a cassette system similar to the cassette system disclosed in WO 2008/053259. In one embodiment, the cassette system also comprises additional functional parts such as pressure transducers **(21,22),** pump segments **(25,27,28),** and valves **(23,24).** Such a cassette system is a single use module including wear parts. It is easy to handle and greatly reduces the need for maintenance of the system, as wear parts of the system are routinely exchanged with the single use module. It also provides improved safety for the patient, as it provides a closed sterile system. Additionally, the risk of contamination or the formation of microbial plaques in parts with complex geometries and flow paths is eliminated, when these are integrated into the single use cassette system.

The cassette system can be expanded by a module comprising a positively charged microporous filter membrane, said module being directly incorporated into the cassette. By incorporating the filter membrane into the cassette, the filtration system is simplified and on-line preparation of medical fluid can be conducted automatically after mounting the cassette onto the machine and initialising the process.

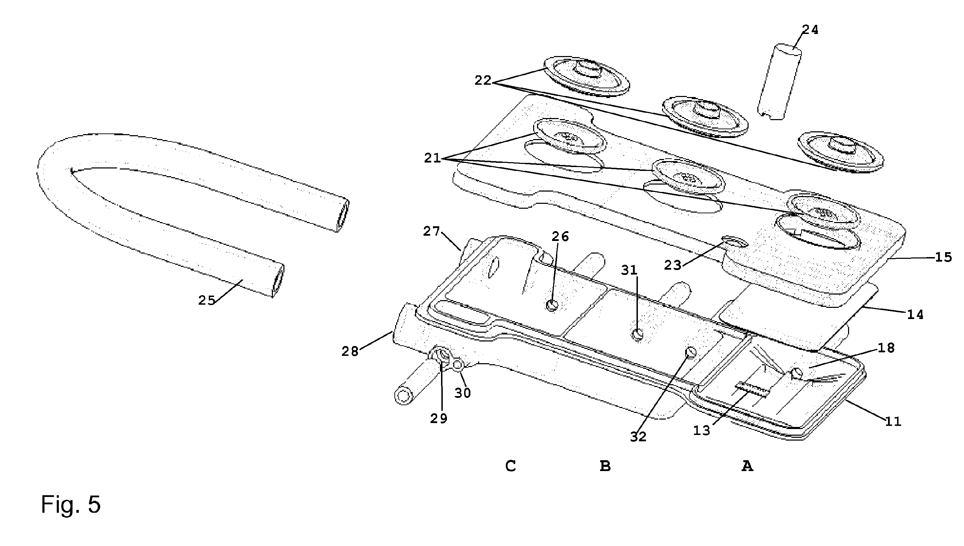

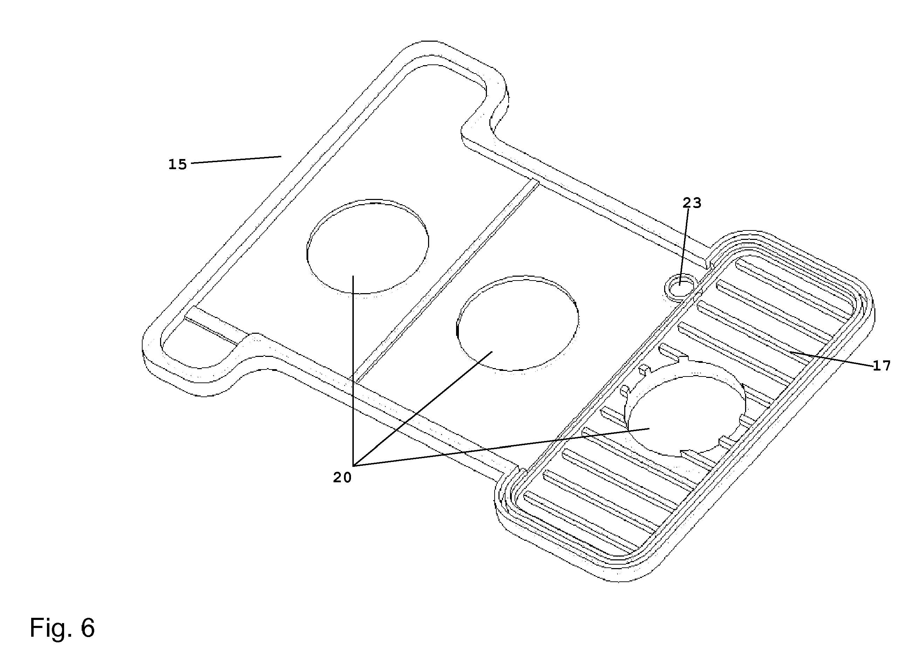

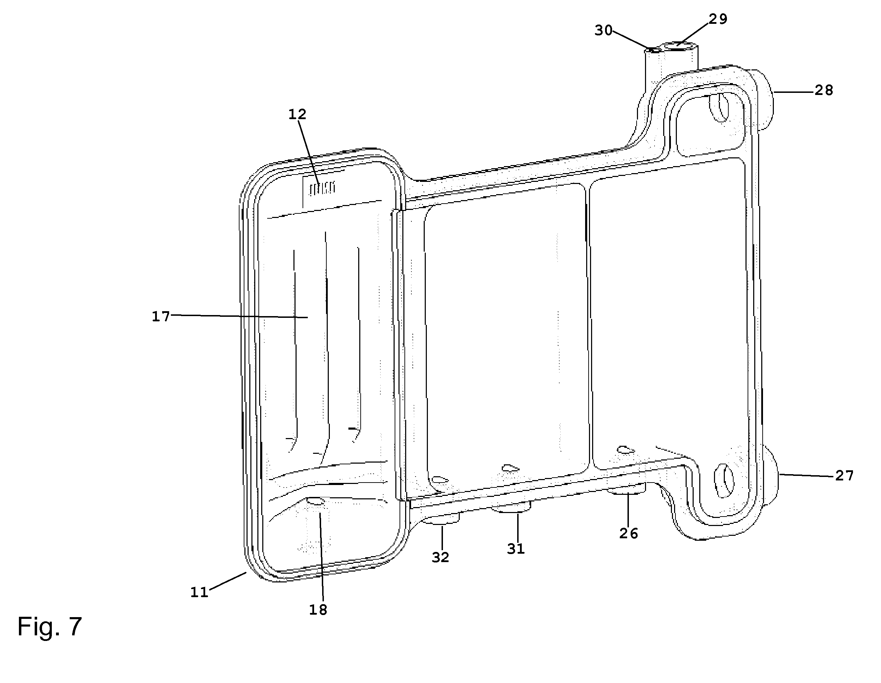

An exemplary embodiment of a single use module according to the invention which incorporates the functions of a cassette system is shown in Figs. 5 to 7. The module comprises a filter unit **(A),** venous **(B)** and arterial **(C)** expansion compartments, pressure transducers **(21,22)** comprising a first **(21)** and a second **(22)** impermeable diaphragm, a pump tube tract **(25),** and a valve **(23,24).** Base plate **(15)** comprises assembly openings **(20)** for pressure transducers **(21,22),** a membrane support structure **(17)** which also guides the flow towards the valve **(23, 24)** which separates the filter compartment **(A)** from the venous expansion chamber **(B).The** housing cover **(11)** comprises inlets **(18,26,28,30,32)** and outlets **(27,29,31)** for fluids. It additionally comprises venting slots **(12)** equipped with a deaeration membrane **(13)** and a membrane support structure **(17).** The medical fluid enters the filter compartment **(A)** through inlet **(18)** and is filtered through filtration membrane **(14).** Any air entrapped in the fluid passes deaeration membrane **(13)** and is purged through the venting slots **(12).** When the valve **(23,24)** formed by valve chamber **(23)** and notched pin **(24)** is opened, the fluid enters into the venous expansion compartment **(B).** Blood coming out of the dialyzer is fed to the venous expansion compartment **(B)** via inlet **(32),** intermixes with the medical fluid, leaves through outlet **(31)** and is pumped back into the patient. Arterial blood coming out of the patient's vascular access is fed to the arterial expansion compartment **(C)** via inlet **(26),** enters the pump tube tract **(25)** through outlet **(31),** is pumped into the upper chamber of the arterial expansion compartment **(C)** through inlet **(28)** and discharged through outlet **(29)** and then into the dialyzer. A service line **(30)** is provided to permit the addition of fluids, for instance anticoagulants, to the arterial blood.

An advantage of single use modules is a further improvement of patient safety as it serves as sterile barrier before the fluid out of the filter enters the patient's blood.

In one embodiment, the single use filtration module of the invention comprises a hydrophilic positively charged flat sheet microporous membrane **(14)**. In one embodiment, the membrane has a thickness of 100 to 180 µm, for instance 100-140 µm, a pore size of about 0.2 µm (e.g. from 0.18 to 0.22 µm), a surface area of 8 to 30 cm², for instance 15 to 25 cm², in accordance with the flow required in conjunction with the pressure drop.

If the surface area is smaller than 8 cm², retention of cytokine inducing substances (CIS) might be insufficient. If the surface area is larger than 30 cm², the risk of an uneven flow distribution becomes large and the design of a support structure, which is critical for the membrane, is difficult for high flow rates. The membrane surface area has to be selected in order to guarantee a sterility assurance level (SAL) of 6 for the medical fluid.

In one embodiment, the medical fluid delivery system according to the invention provides medical fluid at a rate of 0 to 450 ml/min. In comparison, commercially available systems only achieve rates of about 30 ml/min. The system can provide a total volume of 150 1 of medical fluid, which is the maximum fluid volume reported for pre-/post- hemodiafiltration and hemofiltration. This fluid volume can be reached in 5-6 hrs.

The housing of the module is made of a polymer material which is biocompatible and suitable for human use and fulfils manufacturer's product specifications. For optical control and for visual control, e.g. for air traps, during treatment, the housing preferably is transparent. Examples of suitable polymer materials are polycarbonate (PC), polyethylene terephthalate (PETG), and polyurethane (PUR).

The housing has a vent opening **(12)** equipped with a hydrophobic deaeration membrane **(13)** for automatically removing air from the system during operation and avoiding pressure oscillation within the system. A suitable material for the deaeration membrane **(13)** is polytetrafluoroethylene (PTFE). The position of the vent opening **(12)** and the deaeration membrane **(13)** is chosen to facilitate air removal during treatment, i.e. it is preferably positioned in a part of the housing which is located near the top of the module during operation. The size of the deaeration membrane **(13)** has to be selected according to the air volume which has to be removed from the medical fluid delivery system.

Several technologies can be employed to join and seal the two parts **(11,15)** of the housing. Examples of useable sealing technologies are heat sealing, heat contact welding, laser welding, ultrasonic welding, RF welding, or friction welding.

The design and location of the inlet **(18)** and the outlet **(19)** is dependent on both flow distribution desired and production technology to be used. For instance, using a laser welding process to join and seal the two parts **(11,15)** of the housing would require planar surfaces for welding and laser light adsorption.

The membrane support structure **(17)** serves two purposes: it supports the filtration membrane **(14)** during filtration in order to avoid stretching of the membrane due to pressure differentials and other causes, and it ensures an even flow distribution and smooth discharge of the liquid from the filter.

In one embodiment, the filter membrane **(14)** is sealed into the housing of the filter module by heat sealing or heat contact welding. During the sealing process, the polymer material of the housing melts and penetrates into the filter membrane **(14)** to provide an additional seal. Due to its pore size, a microporous filter membrane allows for the permeation of the polymer melt, while the pores of an ultrafilter membrane would be too small. In one embodiment, the deaeration membrane **(13)** is sealed into the housing by ultrasound welding. In another embodiment, the membranes **(13,14)** are clamped into the housing. In still another embodiment, bonding techniques using materials such as UV bonding materials, are employed to affix the membranes **(13,14)** to the housing.

In another aspect of the present disclosure that is not part of the claimed invention, the medical fluid delivery system can perform special fluid delivery functions, such as a prime, a bolus infusion and a blood rinse-back using fluid components in an efficient arrangement.

Those functions can be commenced manually or automatically, e.g., upon receipt of a signal from a suitable biosensor. In one example involving HF/HDF, a two-way valve is placed in the post dialyzer therapy fluid or dialysate circuit.

In one implementation, the valve is used to perform a bolus infusion, e.g., to stabilize the patient who has low blood pressure or is hypotensive. The bolus amount can be predetermined or entered at the time it is needed. Upon an operator input or suitable signal from a sensor, a bypass valve in the upstream dialysate line is closed so that normal flow to the dialyzer is stopped. A valve located downstream of the dialyzer is also closed, so that the dialyzer is separated from the bloodstream which is flowing through the bypass. The fluid is infused into the bypass and enters the bloodstream of the patient. After the bolus amount is delivered, the valve is closed and the patient's blood pressure is allowed to stabilize and then normal therapy is resumed. The amount of the bolus volume is either predetermined or set by the operator upon initiating the bolus function.

The above system is also suitable to perform a substitution fluid rinse-back at the end of therapy to rinse blood remaining in the extracorporeal circuit back to the patient. The rinse-back feature, like the bolus volume, can be initiated automatically. An amount of rinse-back solution can be preset or set at the time of the procedure. The substitution fluid pump delivers the programmed rinse-back amount to rinse-back the extracorporeal circuit into the patient. The machine is set to alert the operator when the rinse-back is complete.

Obviously, the procedures are performed at different times during therapy because the different procedures are for different purposes. The bolus function as described above is initiated manually or automatically when the patient appears to have become or is becoming hypotensive. Blood rinse-back is performed at the end of treatment to push any blood remaining in the system back to the patient.

The prime feature operates using the apparatus described above for the bolus and rinse-back features to prime the extracorporeal circuit prior to therapy. The prime includes a volume of fluid, such as dialysate, that is delivered at the beginning of the therapy to remove air from the extracorporeal circuit. The amount or volume can be predetermined prior to commencement of therapy. Alternatively, the amount or volume is delivered until air is no longer present in the extracorporeal circuit.

It will be understood that the features mentioned above and those described hereinafter can be used not only in the combination specified but also in other combinations or on their own, without departing from the scope of the present invention.

The present invention will now be described in more detail in the examples below. It is to be understood that the examples are not intended to limit the scope of the present invention and are merely an illustration of a preferred embodiment of the invention.

### Examples

The aptitude of different filter configurations for the production of sterile medical fluid which is free from cytokine inducing substances was tested. Solutions of ssDNA were used as a model system for medical fluids and retention of ssDNA within the system was determined. The ssDNA used was 5' TCg ACT CTC gAg CgT TCT T; 19-mer, MW: 5750 Da (TIB MOLBIOL GmbH, 12103 Berlin, Germany).

To prepare the test solution, Dialysis Concentrate (D242 10 L, Gambro) and Bicarbonate Hemodialysis Concentrate (D200 10 L, Gambro) were added to RO water. The solution was heated to 37°C and filtered with an U8000S ultrafilter (Gambro). A stock solution containing ssDNA was added and the concentration of ssDNA in the test solution was adjusted to 1,000 ng/ml.

The respective filter device was perfused with the test solution at 37°C using the flow rate indicated in the individual working example. Samples of the filtrate were collected periodically and the concentration of ssDNA in the filtrate was determined using an OliGreen^{®} ssDNA Quantitation Kit (Molecular Probes, Inc., Eugene, Oregon, USA).

### Example 1

Retention of ssDNA by a positively charged microporous flat sheet membrane (Supor^{®} HP-200, Pall Corporation, Port Washington, NY, USA) having a pore size of 0.2 µm was tested by dead-end filtration of the test solution.
a) 1,000 ml of the test solution were filtered at a flow rate of 20 ml/min through a membrane having a surface area of 9.8 cm². Samples of the filtrate were taken after filtration of 100 ml, 250 ml, 500 ml, 750 ml, 1,000 ml, and from the filtrate pool. The concentration of ssDNA in all samples was below the detection limit of 1,250 pg/ml.
b) 1,100 ml of the test solution were filtered at a flow rate of 450 ml/min through a membrane having a surface area of 25 cm². Samples of the filtrate were taken after filtration of 500 ml, 1,000 ml, and from the filtrate pool. The concentration of ssDNA in all samples was below the detection limit of 1,250 pg/ml.

### Example 2 (comparative)

Retention of ssDNA by an uncharged microporous flat sheet membrane (Supor^{®} 200, Pall Corporation, Port Washington, NY, USA) having a pore size of 0.2 µm and a surface area of 9.8 cm² was tested by dead-end filtration of 1,000 ml of the test solution at a flow of 20 ml/min. Samples of the filtrate were taken after filtration of 100 ml, 250 ml, 500 ml, 750 ml, 1,000 ml, and from the filtrate pool. The concentration of ssDNA in all samples was equal to the concentration of ssDNA in the test solution.

### Example 3 (comparative)

Retention of ssDNA in a mini-module containing positively charged hollow fiber ultrafiltration membranes having a total surface area of 360 cm² was tested by dead-end filtration of the test solution.

3,000 ml of the test solution were filtered at a flow rate of 14 ml/min. Samples of the filtrate were taken after filtration of 500 ml, 1,000 ml, 1,500 ml, 2,000 ml, 2,500 ml, 3,000 ml, and from the filtrate pool. Concentration of ssDNA in the samples was determined to be 3.1 ng/ml, 3.5 ng/ml, 2.4 ng/ml, 2.8 ng/ml, 2.1 ng/ml, 2.6 ng/ml, and 2.1 ng/ml, respectively.

## Claims

1. A medical fluid delivery system which is capable of on-line production of a medical fluid which is sterile, non-pyrogenic, and free from cytokine-inducing substances, and capable of providing medical fluid at a rate of 0 to 450 ml/min, comprising at least two filter modules (**2,3,4**) connected in series, at least one filter module comprising an ultrafilter and at least one filter module comprising a microporous filter, **characterised in that** the final filter module in the series comprises a microporous filter, wherein the microporous filter comprises a positively charged microporous flat sheet membrane (**14**) having a surface area in the range of from 8 to 30 cm².

2. The system of claim 1, wherein the positively charged microporous membrane has a thickness of 100 to 180 µm.

3. The system of claims 1 or 2, wherein the positively charged microporous membrane has a pore size of from 0.18 to 0.22 µm.

4. The system of any one of claims 1 to 3, wherein the positively charged microporous membrane comprises polyethersulfone.

5. The system of any one of claims 1 to 4, wherein the filter module comprising a microporous filter is a single use filtration module.

6. The system of claim 5, wherein the single use filtration module comprises a housing **(11,15),** a positively charged microporous flat sheet membrane **(14)** arranged within the housing, and a vent opening **(12)** equipped with a deaeration membrane **(13).**

7. The system of claim 6, wherein the inlet **(18)** and the outlet **(19)** of the single use filtration module are located on the same side of the housing **(11,15).**

8. The system of claim 6, wherein the inlet **(18)** and the outlet **(19)** of the single use filtration module are located on opposite sides of the housing **(11,15).**

9. The system of any one of claims 5 to 8, wherein the single use filtration module further comprises valves **(23,24).**

10. The system of any one of claims 5 to 9, wherein the single use filtration module further comprises pressure transducers **(21,22).**

11. The system of any one of claims 5 to 10, wherein the single use filtration module further comprises pump segments **(25,27,28).**

12. The system of any one of claims 5 to 11, wherein the single use filtration module further comprises venous **(B)** and arterial **(C)** expansion compartments.

13. A method of using the system of claim 1 for producing a sterile medical fluid free from bacteria, endotoxins and cytokine inducing substances, comprising the steps of filtering the medical fluid through an ultrafiltration membrane and subsequently through a positively charged microporous membrane.

## Patentansprüche

1. System zur Abgabe medizinischer Flüssigkeit, welches in der Lage ist, eine medizinische Flüssigkeit, die steril, nicht pyrogen, und von Bakterien, Endotoxinen, und Cytokininduzierenden Substanzen frei ist, online herzustellen und medizinische Flüssigkeit mit einer Rate von 0 bis 450 ml/min bereitzustellen, umfassend mindestens zwei hintereinander geschaltete Filtermodule **(2,3,4)**, wobei mindestens ein Filtermodul einen Ultrafilter und mindestens ein Filtermodul einen mikroporösen Filter umfasst, **gekennzeichnet dadurch, dass** das letzte Filtermodul in der Reihe einen mikroporösen Filter umfasst, wobei der mikroporöse Filter eine positiv geladene mikroporöse Flachmembran (**14**) mit einer Oberfläche im Bereich 8 bis 30 cm² umfasst.

2. System gemäß Anspruch 1, worin die positiv geladene mikroporöse Membran eine Dicke von 100 bis 180 µm aufweist.

3. System gemäß Anspruch 1 oder 2, worin die positiv geladene mikroporöse Membran eine Porengröße von 0,18 bis 0,22 µm aufweist.

4. System gemäß einem der Ansprüche 1 bis 3, worin die positiv geladene mikroporöse Membran Polyethersulfon enthält.

5. System gemäß einem der Ansprüche 1 bis 4, worin das einen mikroporösen Filter umfassende Filtermodul ein Einweg-Filtrationsmodul ist.

6. System gemäß Anspruch 5, worin das Einweg-Filtrationsmodul ein Gehäuse **(11,15)**, eine in dem Gehäuse angeordnete positiv geladene mikroporöse Flachmembran **(14)** und eine mit einer Entlüftungsmembran **(13)** ausgestattete Entlüftungsöffnung **(12)** umfasst.

7. System gemäß Anspruch 6, worin der Einlass **(18)** und der Auslass **(19)** des Einweg-Filtrationsmoduls sich auf derselben Seite des Gehäuses **(11,15)** befinden.

8. System gemäß Anspruch 6, worin der Einlass **(18)** und der Auslass **(19)** des Einweg-Filtrationsmoduls sich auf gegenüberliegenden Seiten des Gehäuses **(11,15)** befinden.

9. System gemäß einem der Ansprüche 5 bis 8, worin das Einweg-Filtrationsmodul ferner Ventile **(23,24)** umfasst.

10. System gemäß einem der Ansprüche 5 bis 9, worin das Einweg-Filtrationsmodul ferner Druckwandler **(21,22)** umfasst.

11. System gemäß einem der Ansprüche 5 bis 10, worin das Einweg-Filtrationsmodul ferner Pumpensegmente **(25,27,28)** umfasst.

12. System gemäß einem der Ansprüche 5 bis 11, worin das Einweg-Filtrationsmodul ferner venöse **(B)** und arterielle **(C)** Expansionskammern umfasst.

13. Verfahren zur Verwendung des Systems gemäß Anspruch 1 zur Herstellung einer von Bakterien, Endotoxinen, und Cytokin-induzierenden Substanzen freien sterilen medizinischen Flüssigkeit, umfassend die Schritte des Filtrierens der medizinischen Flüssigkeit durch eine Ultrafiltrationsmembran und anschließend durch eine positiv geladene mikroporöse Membran.

## Revendications

1. Système de distribution de fluide médical qui est capable de production en ligne d'un fluide médical qui est stérile, non pyrogène, et exempt de substances induisant des cytokines, et capable de fournir le fluide médical à un débit de 0 à 450 ml/min, comprenant au moins deux modules de filtre (2, 3, 4) connectés en série, au moins un module de filtre comprenant un ultrafiltre et au moins un module de filtre comprenant un filtre microporeux, **caractérisé en ce que** le module de filtre final dans la série comprend un filtre microporeux, dans lequel le filtre microporeux comprend une membrane en feuille plane microporeuse (14) chargée positivement ayant une aire de surface dans la plage de 8 à 30 cm².

2. Système selon la revendication 1, dans lequel la membrane microporeuse positivement chargée a une épaisseur de 100 à 180 µm.

3. Système selon les revendications 1 ou 2, dans lequel la membrane microporeuse positivement chargée a une taille de pores de 0,18 à 0,22 µm.

4. Système selon l'une quelconque des revendications 1 à 3, dans lequel la membrane microporeuse positivement chargée comprend du polyéthersulfone.

5. Système selon l'une quelconque des revendications 1 à 4, dans lequel le module de filtre comprenant un filtre microporeux est un module de filtration à usage unique.

6. Système selon la revendication 5, dans lequel le module de filtration à usage unique comprend un boîtier (11, 15), une membrane en feuille plane microporeuse (14) chargée positivement et agencée dans le boîtier, et une ouverture d'évent (12) équipé d'une membrane de désaération (13).

7. Système selon la revendication 6, dans lequel l'entrée (18) et la sortie (19) du module de filtration à usage unique sont situées sur le même côté du boîtier (11, 15).

8. Système selon la revendication 6, dans lequel l'entrée (18) et la sortie (19) du module de filtration à usage unique sont situées sur des côtés opposés du boîtier (11, 15).

9. Système selon l'une quelconque des revendications 5 à 8, dans lequel le module de filtration à usage unique comprend en outre des valves (23, 24).

10. Système selon l'une quelconque des revendications 5 à 9, dans lequel le module de filtration à usage unique comprend en outre des transducteurs de pression (21, 22).

11. Système selon l'une quelconque des revendications 5 à 10, dans lequel le module de filtration à usage unique comprend en outre des segments de pompe (25, 27, 28).

12. Système selon l'une quelconque des revendications 5 à 11, dans lequel le module de filtration à usage unique comprend en outre des compartiments d'expansion veineux (B) et artériels (C).

13. Procédé d'utilisation du système selon la revendication 1 pour produire un fluide médical stérile exempt de bactéries, d'endotoxines et de substances induisant des cytokines, comprenant les étapes consistant à filtrer le fluide médical à travers une membrane d'ultrafiltration et ultérieurement à travers une membrane microporeuse chargée positivement.
